(19) Europäisches Patentamt / European Patent Office / Office européen des brevets

(11) **EP 3 957 306 A1**

(12) **DEMANDE DE BREVET EUROPEEN**

(43) Date de publication:
**23.02.2022   Bulletin 2022/08**

(21) Numéro de dépôt: **21183515.2**

(22) Date de dépôt: **02.07.2021**

(51) Classification Internationale des Brevets (IPC):
*A61K 31/015* (2006.01)     *A61K 31/366* (2006.01)
*A61K 31/7048* (2006.01)     *A61K 31/715* (2006.01)
*A61K 31/724* (2006.01)     *A61K 31/728* (2006.01)
*A61P 17/04* (2006.01)     *A61P 17/06* (2006.01)
*A61K 8/60* (2006.01)     *A61Q 19/00* (2006.01)
*A61K 8/73* (2006.01)     *A61Q 17/00* (2006.01)

(52) Classification Coopérative des Brevets (CPC):
(C-Sets disponibles)
A61K 31/366; A61K 8/602; A61K 8/735;
A61K 8/738; A61K 31/015; A61K 31/7048;
A61K 31/715; A61K 31/724; A61K 31/728;
A61P 17/04; A61P 17/06; A61Q 17/00; A61Q 19/00
(Cont.)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité:  **21.08.2020   FR 2008601**

(71) Demandeur: **Purally**
**45100 Orléans (FR)**

(72) Inventeur: **REDZINIAK, Gérard**
**92160 Antony (FR)**

(74) Mandataire: **Demulsant, Xavier**
**Dejade & Biset**
**35, rue de Châteaudun**
**75009 Paris (FR)**

(54) **COMPOSITION COMPRENANT AU MOINS UN FLAVONOIDE GLYCOSYLE ET SON UTILISATION EN COSMETIQUE OU DERMATOLOGIE**

(57)     La présente invention concerne une composition cosmétique et/ou dermatologique comprenant en tant qu'ingrédient actif au moins un flavonoïde glycosylé, caractérisée en ce qu'elle comprend en outre, en tant qu'ingrédient actif un composé capable de limiter ou d'empêcher la pénétration dans les kératinocytes d'un hydrocarbure aromatique polycyclique de type benzo[a]pyrene, cyclopenta[c,d]pyrène, dibenzo[a,h]anthracène ou dibenzo[a,l]pyrène et/ou un composé caractérisé par sa capacité à absorber la lumière bleue.

Fig. 1

EP 3 957 306 A1

**(Cont. page suivante)**

(52) Classification Coopérative des Brevets (CPC):
(Cont.)

C-Sets
**A61K 31/015, A61K 2300/00;**
**A61K 31/366, A61K 2300/00;**
**A61K 31/7048, A61K 2300/00;**
**A61K 31/715, A61K 2300/00;**
**A61K 31/724, A61K 2300/00;**
**A61K 31/728, A61K 2300/00**

**Description**

Domaine technique

La présente invention concerne des compositions comprenant au moins un flavonoïde glycosylé, utilisables en application topique, dans le traitement d'une pathologie non allergique engendrant la production d'IL31, en particulier une pathologie affectant la peau et choisie parmi le prurit, la dermatite atopique, l'eczéma, le psoriasis, le prurigo nodulaire.

Art antérieur

La peau est la principale barrière qui protège le corps humain de l'environnement. Cet environnement est composé de facteurs exogènes qui peuvent engendrer des stress physiques, chimiques ou encore biologiques, qui augmentent le vieillissement cutané et le risque de développer des pathologies cutanées de différents degrés de gravité. Il est désormais admis que la pollution et le rayonnement solaire ont un effet synergique et négatif pour la peau (The skin aging exposome, Krutmann J. et al. Dermatol Sci. 2017 Mar; 85(3):152-161).

La pollution peut prendre différentes formes : pollution atmosphérique, pollution de l'eau, réchauffement climatique (pollution thermique), pollution chimique au travers des industries ou encore des composants d'objets du quotidien (peinture, solvant, colle...).

Le rayonnement solaire composé de lumière visible, UVA/UVB, mais aussi lumière bleue, entraine la production de cytokines inflammatoires de type IL-1, IL-6, IL-8. D'autres études récentes montrent que la lumière bleue, émise par le soleil mais également par tous les éclairages des appareils de type ordinateur, tablette, télévision, téléphone portable, aurait une influence sur le photovieillissement, en augmentant le taux de radicaux libres internes au niveau des kératinocytes et des mélanocytes. Ceci entrainerait aussi la formation de tâche pigmentaire (Duteil et al, Differences in visible light-induced pigmentation according to wavelengths: a clinical and histological study in comparison with UVB exposure, Pigment Cell Melanoma Res, 2014 vol. 27(5) pp. 822-6 T).

La pollution atmosphérique joue également un rôle important dans les pathologies de la peau, et notamment les hydrocarbures aromatiques polycycliques (HAP) qui proviennent de la combustion du bois, mais surtout en milieu urbain des gaz d'échappement. Dans une étude récente, des chercheurs ont montré, sur des explants de peaux humaines, que la métabolisation des HAP entraine la production de métabolites facilement éliminés, mais qu'une petite fraction réagit avec l'ADN pour induire des mutations. Cette étude montre également que l'exposition aux UV solaires diminue la capacité des cellules cutanées à métaboliser les HAP, qui pourraient ainsi s'accumuler dans les tissus (Metabolism and genotoxicity of polycyclic aromatic hydrocarbons in human skin explants: mixture effects and modulation by sunlight, Anne von Koschembahr et al., Archives of Toxicology volume 94, pages 495-507(2020) ; Solar simulated light exposure alters metabolization and genotoxicity induced by benzo[a]pyrene in human skin, Anne von Koschembahr et al., Scientific Reports volume 8, Article number: 14692 (2018)).

La diminution du métabolisme pourrait entraîner une accumulation de benzo[a]pyrene (B[a]P) et une toxicité à long terme.

Certaines pathologies de la peau ne présentent pas de composante inflammatoire, c'est le cas du prurit par exemple, qui néanmoins est caractérisé par la sécrétion de certaines cytokines comme IL-31 (Interleukine 31) (IL-31 is crucial for induction of pruritus, but not inflammation, in contact hypersensitivity, Ayako Takamori et al., Sci Rep (2018) 8:6639).

L'IL-31 est une cytokine de la famille de l'IL-6, produit pas les lymphocytes T CD4+ activés, suggérant son implication dans le développement d'une réponse immune de type Th2. Il a été démontré que des souris transgéniques pour IL-31 développent des peaux inflammatoires de type dermatite atopique. Par ailleurs, Il-31 semble responsable des prurits.

Il existe différentes manières de traiter les démangeaisons, en particulier les démangeaisons induites par une augmentation de la production d'IL-31. Des anticorps thérapeutiques anti-IL-31 ont été développés et sont utilisés dans le traitement du prurit ou de la dermatite atopique comme le nemolizumab, anticorps développé par la société Galderma en essais cliniques dans la dermatite atopique modérée à sévère. D'autres anticorps anti-IL-31 ont été décrits (US10273297, EP2734549), pour leur utilisation dans ce champ d'application. Néanmoins, les traitements par anticorps thérapeutiques ne sont pas sans effet secondaire, puisqu'ils ne sont pas spécifiques de l'IL-31 produite par les kératinocytes de la peau. Par ailleurs, ces traitements sont couteux. Ainsi, il existe un réel besoin d'une solution permettant d'atténuer la production de l'IL-31 dans les kératinocytes par un traitement au niveau local.

Les inventeurs ont d'abord observé que la production d'IL-31 par des kératinocytes humains était augmentée après une exposition à des HAP en synergie avec une exposition à de la lumière bleue.

Les inventeurs ont, de manière surprenante, mis en évidence que des composés de type flavonoïdes glycosylés permettent de diminuer la production d'IL-31 par les kératinocytes, induite par l'exposition aux HAP et à la lumière bleue.

Problème technique à résoudre

Un but de la présente invention est de fournir des compositions cosmétiques et/ou dermatologiques permettant de diminuer la production d'IL-31 au niveau de la peau et de traiter ou prévenir une pathologie affectant la peau, choisie parmi le prurit, la dermatite atopique, l'eczéma, le psoriasis, le prurigo nodulaire.

Description détaillée de l'invention

Un objet de la présente invention est de fournir une composition cosmétique et/ou dermatologique comprenant en tant qu'ingrédient actif au moins un flavonoïde glycosylé, caractérisée en ce qu'elle comprend en outre, en tant qu'ingrédient actif, un composé capable de limiter ou d'empêcher la pénétration dans les kératinocytes d'un hydrocarbure aromatique polycyclique de type benzo[a]pyrene, cyclopenta[c,d]pyrène, dibenzo[a,h]anthracène ou dibenzo[a,l]pyrène et/ou un composé caractérisé par sa capacité à absorber la lumière bleue.

On entend par « composition cosmétique », toute substance ou composition destinée à être mise en contact avec les diverses parties superficielles du corps humain (épiderme, systèmes pileux et capillaire, ongles, lèvres et organes génitaux externes) ou avec les dents et les muqueuses buccales, en vue, exclusivement ou principalement, de les nettoyer, de les parfumer, d'en modifier l'aspect, de les protéger, de les maintenir en bon état ou de corriger les odeurs corporelles.

On entend par « composition dermatologique », toute substance ou composition destinée à traiter ou prévenir les affections de la peau ou pour les soins réguliers de la peau.

On entend par « ingrédient actif », une substance naturelle ou chimique, extraite ou synthétique, entrant dans la formulation d'une composition thérapeutique ou cosmétique, caractérisée en ce qu'elle possède un effet bénéfique, de traitement ou de prévention sur une pathologie ou un symptôme.

On entend par « flavonoïde », tout composé appartement à la famille des flavonoïdes, caractérisé par une même structure de base formée par deux cycles aromatiques reliés par trois carbones : C6-C3-C6, réparties en classes que sont les flavones, les flavonols, les flavononols ou dihydroflavonols, les flavanones, les aurones, les chalcones et les dihydrochalcones.

On entend par « flavonoïde glycosylé », tout composé appartenant aux classes décrites ci-dessus, portant au moins un sucre choisi parmi le glucose, le galactose, le rhamnose. Les flavonoïdes selon l'invention sont des flavonoïdes naturels ou synthétiques. Les flavonoïdes sont naturellement présents dans les fruits, les légumes, le thé, le vin, mais aussi dans certaines plantes médicinales comme l'aubépine, le ginkgo, la passiflore, la menthe poivrée, l'artichaut, ou encore le margousier, le goyabier, mais aussi beaucoup d'autres plantes et fleurs (liste non exhaustive). Il est possible de les extraire. De nombreux protocoles ont été publiés et sont accessibles à l'homme du métier.

Dans un mode de réalisation de l'invention, la composition selon l'invention comprend en outre, en tant qu'ingrédient actif, un composé capable de limiter ou d'empêcher la pénétration dans les kératinocytes d'un hydrocarbure aromatique polycyclique de type benzo[a]pyrene, cyclopenta[c,d]pyrène, dibenzo[a,h]anthracène ou dibenzo[a,l]pyrène.

On entend par « limiter ou empêcher la pénétration dans les kératinocytes d'un hydrocarbure aromatique polycyclique de type benzo[a]pyrène, cyclopenta[c,d]pyrène, dibenzo[a,h]anthracène ou dibenzo[a,l]pyrène », la capacité de toute substance à maintenir à la surface externe de la peau, les agents polluants de type HAP. En d'autres termes, limiter ou empêcher la pénétration dans les kératinocytes d'un HAP, revient à maintenir les kératinocytes dans leur état d'équilibre physiologique, c'est-à-dire lorsque la structure et les fonctions ne sont pas altérées, qu'ils ne présentent pas de caractéristiques ou de marqueurs d'inflammation comme la synthèse et la libération de cytokines comme le TNF alpha par exemple (The influence of PM2.5 on lung injury and cytokines in mice. Yang J, et al., Exp Ther Med. 2019 Oct,18(4):2503-2511). Cette interleukine extrêmement agressive provoque des dégradations membranaires et cellulaires cutanées responsables de l'inflammation et du vieillissement de la peau (Wang Y., et al., Mech Ageing Dev. 2019 Dec, 184:111160). Les hydrocarbures aromatiques polycycliques aussi appelés HAP, sont de type benzo[a]pyrene ($C_{20}H_{12}$), cyclopenta[c,d]pyrène ($C_{18}H_{10}$), dibenzo[a,h]anthracène ($C_{22}H_{14}$) ou dibenzo[a,l]pyrène ($C_{24}H_{18}$ or $C_{24}H_{14}$) ou des dérivés de ces composés. Ces composés sont générés par la combustion de matières fossiles (notamment par les moteurs diesels) sous forme gazeuse ou particulaire. Les HAP sont donc notamment présents dans la pollution aérienne urbaine.

Le passage des hydrocarbures dans l'organisme humain s'effectue par inhalation, par ingestion, mais également par transfert au travers de la peau. En effet, les inventeurs ont montré dans des travaux précédents cette invention, que l'exposition de kératinocytes humains à des HAP augmentait la production d'IL-31 au niveau des kératinocytes.

Dans un mode de réalisation de l'invention, la composition selon l'invention peut aussi comprendre un composé caractérisé par sa capacité à absorber la lumière bleue.

On entend par « capacité à absorber la lumière bleue », la capacité de toute substance à prévenir les effets négatifs de la lumière bleue sur le vieillissement cutané et les altérations de la peau, en limitant l'exposition de la peau à ladite lumière bleue.

La lumière bleue située entre 400 et 500 nanomètres (nm) de longueurs d'onde fait partie du spectre de la lumière visible qui est compris entre 400 et 800 nm. Elle est émise par l'irradiation solaire mais également par tous les appareils électroniques disposant d'un écran à base de Diodes Electro-Luminescentes (aussi appelées LED) comme les smartphones, les tablettes, les télévisions ou les ordinateurs.

L'énergie émise par cette lumière bleue peut être voisine de 40 J/cm$^2$ pour l'irradiation solaire et de 10J/cm$^2$ pour les écrans des appareils électroniques.

Dans un mode de réalisation préféré, le flavonoïde glycosylé est un flavonol glycosylé. Les flavonols constituent une classe de flavonoïdes caractérisés par une fonction phénol en C3 et une fonction carbonyle C=O en C4 sur l'hétérocycle central du squelette de base des flavonoïdes. Les flavonols glycosylés peuvent être sous la forme de mono, di ou triglycosides. Parmi les monoglycosides, se trouve l'astragaline (kaempferol contenant un glucose en R3), l'azaléine (azaléatine contenant un rhamnose en R3), l'hyperoside (quercétine contenant un galactose en R3), l'isoquercitine (quercétine contenant un glucose en R3), la myricitrine (myricétine contenant un rhamnose en R3), la quercitrine (quercétine contenant un rhamnose en R3), la rutoside (quercétine contenant un rutinose en R3), la xanthorhamnine (rhamnétine contenant un triholoside en R3), l'amurensine (kaempférol contenant un glucose en R7 et un tert-amyl alcool en R8), la spiraéoside (quercétine contenant un glucose en R4') et la troxerutine (quercétine contenant un rutinose en R3 et 3 hydroxyéthyle).

Parmi les diglycosides, se trouve, la kaempferitrine (kaempférol contenant un rhamnose en R3 et un rhamnose en R7), la robinine (kaempférol contenant un robinose en R3 et un rhamnose en R7), l'icariine (kaempféride contenant un rhamnose en R3 et un glucose en R4 et un groupement prényl et un groupement méthoxyle). Dans un mode de réalisation préféré, le flavonoïde glycosylé est rhamnosylé ou glucosylé de préférence rhamnosylé.

Dans un mode de réalisation préféré, le flavonoïde rhamnosylé est choisi parmi la myricitrine (5,7-dihydroxy-3-[(2S,3R,4R,5R,6S)-3,4,5-trihydroxy-6-methyloxan-2-yl]oxy-2-(3,4,5-trihydroxyphenyl)chromen-4-one), la quercitrine (2-(3,4-Dihydroxyphenyl)-5,7- dihydroxy-3-[ (2S,3R,4R,5R,6S)- 3,4,5-trihydroxy-6-methyl-2-tetrahydropyranyl]oxy]-4-chromenone), la kaempferitrine ($C_{27}H_{30}O_{14}$), l'azaléine (2-(3,4-dihydroxyphényl)-7-hydroxy-5-méthoxy-3-[(2S,3R,4R,5R,6S)-3,4,5-trihydroxy-6-méthyloxan-2-yl]oxychromèn-4-one), l'icariine (5-Hydroxy-2-(4-methoxyphenyl)-8-(3-methylbut-2-enyl)-7-[(2S,3R,4S,5S,6R)-3,4,5-trihydroxy-6-(hydroxymethyl)oxan-2-yl]oxy-3-[(2S,3R,4R,5R,6S)-3,4,5-trihydroxy-6-methyloxan-2-yl]oxychromen-4-one) ou un mélange d'au moins deux de ces composés. Le mélange peut être constitué de myricitrine et de quercitrine, ou de myricitrine et de kaempferitrine ou de myricitrine et d'azaléine, ou de myricitrine et d'icariine, ou de quercitrine et d'azaléine, ou de quercitrine et icariine, ou de azaléine et icariine. Le mélange peut aussi être constitué de trois flavonoïdes rhamnosylés tel que la myricitrine, la quercitrine et l'azaléine, ou la myricitrine, la quercitrine et l'icariine, ou la myricitrine, la kaempferitrine et l'azaléine, ou la myricitrine, l'azaléine et l'icariine ou la quercitrine, l'azaléine et l'icariine par exemple. La liste n'est pas limitative.

Dans un mode de réalisation préféré, le flavonoïde rhamnosylé est la myricitrine. Dans un mode de réalisation de l'invention, le composé capable de limiter ou d'empêcher la pénétration dans les kératinocytes d'un hydrocarbure aromatique polycyclique de type benzo[a]pyrène, cyclopenta[c,d]pyrène, dibenzo[a,h]anthracène ou dibenzo[a,l]pyrène est choisi parmi les polysaccharides, les polysaccharides cycliques de type cyclodextrine, les émulsifiants, l'acide hyaluronique lipophilisé.

La peau est constituée de différentes couches et de différents types cellulaires. La couche la plus externe, l'épiderme, est constituée de kératinocytes, de mélanocytes et de cellules de Langherans. Cet épiderme se décompose en quatre couches, caractérisées par le niveau de maturité des kératinocytes qui les composent. De la plus interne à la plus externe, les couches sont : la couche basale, la couche de Malpighi, la couche granuleuse et la couche cornée. Cette couche cornée est une barrière hydrophobe, qui assure fonction de barrière nécessaire à la protection de la peau. Compte tenu du caractère hydrophobe de la couche cornée, les substances lipophiles comme les hydrocarbures aromatiques polycycliques peuvent pénétrer aisément dans les kératinocytes.

Les polysaccharides aussi appelés glycanes, polyosides, polyholosides ou glucides complexes, sont des polymères de la famille des glucides constitués de plusieurs oses liés entre eux par des liaisons osidiques.

Les polysaccharides ont des propriétés hydrophiles. De ce fait, et du fait du caractère hydrophobe des HAP, ces polysaccharides créent une barrière physique hydrophile contre la pénétration de ces substances.

Les polysaccharides utilisés dans le cadre de l'invention peuvent être des polysaccharides cycliques de type cyclodextrine que comptent l'alpha-cyclodextrine ($\alpha$-cyclodextrine, $C_{36}H_{60}O_{30}$), la béta-cyclodextrine ($\beta$-cyclodextrine, $C_{42}H_{70}O_{35}$), la gamma-cyclodextrine (y-cyclodextrine, $C_{48}H_{80}O_{40}$). Les cyclodextrines sont connues pour former une cage moléculaire qui permet d'encapsuler diverses molécules. Elles sont largement utilisées dans les domaines de l'agroalimentaire et pharmaceutique. Leur origine naturelle est un atout. Dans le cadre de la présente invention, les polysaccharides cycliques de type cyclodextrine participent à la capture des HAP par leur structure moléculaire de type « cage hydrophobe » au cœur de la forme cyclique du polymère (6 à 9 résidus) de glucose.

Les cyclodextrines, hydrophobes à l'intérieur, hydrophiles à l'extérieur, capturent les molécules de type HAP et créent un piège moléculaire desdits HAP.

Ainsi, un des objets de l'invention, est une composition comprenant en tant qu'ingrédient actif au moins un flavonoïde glycosylé, plus particulièrement de la myricitrine, de la quercitrine, de l'azaleine, de l'icariine ou un mélange d'au moins deux de ces composés et une cyclodextrine.

Dans le cadre de l'invention, des composés capables de limiter ou d'empêcher la pénétration dans les kératinocytes d'un hydrocarbure aromatique polycyclique sont les émulsifiants. Un émulsifiant est un composé capable de créer une émulsion. Tout émulsifiant possède une extrémité hydrophile et une extrémité hydrophobe, rendant possibles les mélanges de substances hydrophiles et hydrophobes. Les hydrocarbures aromatiques polycycliques sont par nature, lipophiles et hydrophobes.

L'ajout dans la composition de l'invention, d'un composé de type cyclodextrine, vise à créer une émulsion entre un HAP ou un mélange d'HAP et un composé hydrophile, afin de limiter ou d'empêcher la pénétration dans les kératinocytes desdits HAP.

Les émulsifiants utilisables dans le cadre de la composition de l'invention sont : la lécithine, les phospholipides, la lanoline ou la cire d'abeilles (émulsifiants naturels), le stéarate de glycérol, le polysorbate 60 et le mélange de PEG-6/PEG32/Glycol Stéarate vendu sous la dénomination de Tefose® 63, des glycolipides de type sophorolipides ou l'octylglucoside des saponines, des lipoprotéines (liste non exhaustive et non limitative).

Ainsi, un des objets de l'invention, est une composition comprenant en tant qu'ingrédient actif au moins un flavonoïde glycosylé, plus particulièrement de la myricitrine, de la quercitrine, de l'azaleine, de l'icariine ou un mélange d'au moins deux de ces composés et un émulsifiant.

Dans le cadre de l'invention, un composé capable de limiter ou d'empêcher la pénétration dans les kératinocytes d'un hydrocarbure aromatique polycyclique est aussi de l'acide hyaluronique lipophilisé.

L'acide hyaluronique lipophilisé a des propriétés amphiphiles lipophiles et hydrophiles comme une base émulsionnante. Cette molécule capte les molécules lipophiles comme le B[a]P au niveau des chaînes grasses qui constituent la partie lipophilisée. Ainsi, un des objets de l'invention, est une composition comprenant en tant qu'ingrédient actif au moins un flavonoïde glycosylé, plus particulièrement de la myricitrine, de la quercitrine, de l'azaleine, de l'icariine ou un mélange d'au moins deux de ces composés et de l'acide hyaluronique lipophilisé.

Dans un mode de réalisation de l'invention, le composé apte à absorber la lumière bleue est choisi parmi les caroténoïdes ou les mélanoïdines.

Les caroténoïdes regroupent les molécules des familles des carotènes et des xanthophylles. Les principaux caroténoïdes sont l'astaxanthine, le lycopène, le béta-carotène, la lutéine et la zeaxanthine. Les caroténoïdes sont liposolubles et ont des propriétés reconnues antioxydantes et de photoprotection. Ainsi, les caroténoïdes peuvent être utilisés dans la composition selon l'invention, pour leur capacité à absorber la lumière bleue (Blue-Violet Light Irradiation Dose Dependently Decreases Carotenoids in Human Skin, Which Indicates the Génération of Free Radicals. Vandersee S. et al., Oxidative Med Cell Longev. Volume 2015, Article ID 579675).

Ainsi, un des objets de l'invention, est une composition comprenant en tant qu'ingrédient actif au moins un flavonoïde glycosylé, plus particulièrement de la myricitrine, de la quercitrine, de l'azaleine, de l'icariine ou un mélange d'au moins deux de ces composés et une cyclodextrine ou un émulsifiant ou de l'acide hyaluronique lipophilisé, et un caroténoïde.

Les mélanoïdines sont des polymères hétérogènes bruns de haut poids moléculaire qui se forment au cours de la réaction de Maillard, par combinaison entre des sucres et des acides aminés. Du fait de leur structure et propriétés chromophores (absorbent dans les longueurs d'ondes de 400 à 500 nm) ils sont de bons filtres à la lumière bleue. (Coffee melanoidins: structures, mechanisms of formation and potential health impacts, Moreira AS, et al., Food Funct. 2012 Sep;3(9):903-15).

Ainsi, un des objets de l'invention est une composition comprenant en tant qu'ingrédient actif au moins un flavonoïde glycosylé, plus particulièrement de la myricitrine, de la quercitrine, de l'azaleine, de l'icariine ou un mélange d'au moins deux de ces composés et une cyclodextrine ou un émulsifiant ou de l'acide hyaluronique lipophilisé, et un composé de la famille des mélanoïdines.

Dans un mode de réalisation de l'invention, la composition de l'invention est formulée pour une application topique.

Les compositions utilisables dans l'invention peuvent se présenter sous toutes les formes galéniques classiquement utilisées pour une application topique et notamment sous forme d'une solution ou suspension aqueuse, alcoolique ou hydroalcoolique ou d'une suspension huileuse ou d'une solution ou d'une dispersion du type lotion ou sérum, d'une émulsion de consistance liquide ou semi-liquide du type lait, obtenues par dispersion d'une phase grasse dans une phase aqueuse (H/E) ou inversement (E/H), ou d'une suspension ou émulsion de consistance molle de type crème (H/E) ou (E/H), ou d'un gel aqueux ou anhydre, d'un onguent, d'une poudre libre ou compactée à utiliser telle quelle ou à incorporer dans un excipient, ou sous forme de microcapsules ou microparticules, ou de dispersions vésiculaires de type ionique et/ou non ionique.

De façon connue, la composition selon l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. L'homme du métier sait choisir les adjuvants et leur quantité selon la forme galénique souhaitée.

Dans un mode de réalisation préférée de l'invention, la composition selon l'invention est formulée sous forme de brume tel que décrit à l'exemple 8.

Dans un mode de réalisation, l'invention concerne un composé flavonoïde glycosylé, de préférence un flavonol rhamnosylé, pour son utilisation dans le traitement d'une pathologie non allergique engendrant la production d'IL31, en particulier une pathologie affectant la peau et choisie parmi le prurit, la dermatite atopique, l'eczéma, le psoriasis, le prurigo nodulaire.

On entend par « traitement », l'atténuation ou la disparition d'au moins un symptôme de la pathologie, pouvant être la diminution de la production d'IL-31 par les kératinocytes, la diminution de la susceptibilité au grattage, la diminution de l'inflammation de la peau et jusqu'à la disparition du ou des symptômes.

Les inventeurs ont montré que l'exposition à des HAP et/ou de la lumière bleue de kératinocytes humains entrainent une augmentation de la production d'IL-31 par lesdits kératinocytes. Par ailleurs, des auteurs ont montré que les démangeaisons cutanées dans le prurit ou la dermatite atopique étaient associées à cette production d'IL-31 (New mechanism underlying IL-31-induced atopic dermatitis, Meng J. el al., J Allergy Clin Immunol. 2018 May;141(5):1677-1689; IL-31: A new key player in dermatology and beyond, Bagci IS et al., J Allergy Clin Immunol. 2018 Mar;141(3):858-866). Un des objets de l'invention concerne un composé flavonoïde glycosylé, de préférence un flavonol rhamnosylé, pour son utilisation dans le traitement d'une pathologie non allergique engendrant la production d'IL-31, en particulier une pathologie affectant la peau et choisie parmi le prurit, la dermatite atopique, l'eczéma, le psoriasis, le prurigo nodulaire.

Le prurit est défini cliniquement comme la sensation subjective et désagréable d'un désir de se gratter. Il y a une composante sensorielle débutant dans la peau, qui transite par les ganglions dorsaux et est traitée dans le système nerveux central. Souvent, la réaction motrice suit sous la forme d'un désir de se gratter. Le fait de se gratter abîme la peau et déclenche une réaction inflammatoire, que renforce encore la démangeaison.

La dermatite atopique, aussi appelée eczéma atopique, est une maladie chronique inflammatoire de la peau. Elle se développe préférentiellement chez le nourrisson et l'enfant, mais peut persister, voir apparaître parfois chez l'adolescent et l'adulte. Elle est caractérisée par une sècheresse cutanée (xérose) associée à des lésions de type eczéma (rougeurs et démangeaisons, vésicules, suintement et croûtes) qui évoluent par poussées.

L'eczéma est un syndrome, ensemble de signes cliniques et de symptômes communs à plusieurs affections, pouvant correspondre à plusieurs maladies inflammatoires de la peau. Ces maladies sont caractérisées par des démangeaisons (prurit), une rougeur congestive de la peau (érythème) et des éruptions cutanées.

Le psoriasis est une maladie inflammatoire chronique caractérisée par des plaques bien délimitées, rouges, en relief, et recouvertes de squames blanchâtres, ou d'une fine pellicule argentée qui se détache facilement.

Le prurigo ou prurigo nodulaire est une dermatose (maladie de peau) caractérisée par des lésions prurigineuses (qui grattent), certaines excoriées et/ou associées à des stries de grattage, accompagnées de démangeaisons féroces pouvant altérer le sommeil. Le grattage entretient les lésions qui deviennent croûteuses. On distingue les prurigos aigus (de durée inférieure à 6 semaines) qui sont le plus souvent secondaires à des morsures d'insectes, des prurigos chroniques qui peuvent être d'origine dermatologique, neurologique, psychiatrique, systémique.

Dans un mode de réalisation préféré de l'invention, le flavonol rhamnosylé utilisé pour le traitement d'une pathologie non allergique engendrant la production d'IL31, en particulier une pathologie affectant la peau et choisie parmi le prurit, la dermatite atopique, l'eczéma, le psoriasis, le prurigo nodulaire, est choisi parmi la myricitrine, quercitrine, la kaempferitrine, l'azaleine, l'icariine ou un mélange d'au moins deux de ces composés. Le mélange peut être constitué de myricitrine et de quercitrine, ou de myricitrine et d'azaléine, ou de myricitrine et d'icariine, ou de quercitrine et d'azaléine, ou de quercitrine et icariine, ou de azaléine et icariine. Le mélange peut aussi être constitué de trois flavonoïdes rhamnosylés tel que la myricitrine, la quercitrine et l'azaléine, ou la myricitrine, la quercitrine et l'icariine, la myricitrine, l'azaléine et l'icariine ou la quercitrine, l'azaléine et l'icariine.

Description des figures

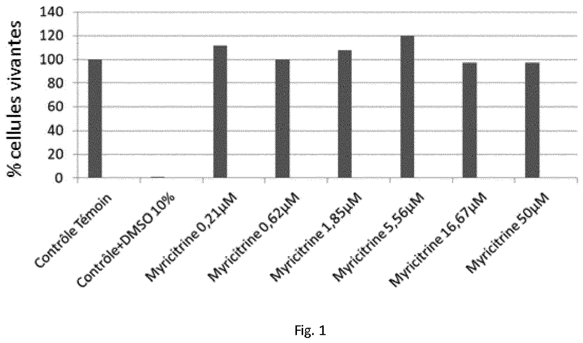

- la figure 1 présente un test de viabilité cellulaire, mesurant le pourcentage de cellules viables, des kératinocytes humains, mis en présence de myricitrine à différentes doses ;

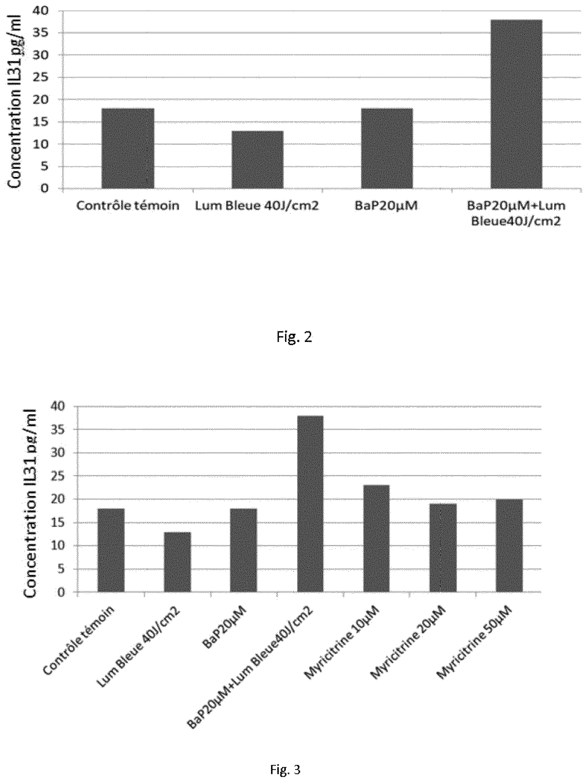

- la figure 2 présente l'effet sur la production d'IL-31 par des kératinocytes humains, de l'alphabenzopyrène, seul ou en association avec de la lumière bleue. L'exposition à la lumière bleue est exprimée en Joule par centimètre carré ($J/cm^2$). L'exposition au alphabenzopyrène est exprimée en micromolaire ($\mu M$) ;
- la figure 3 présente l'effet de la myricitrine sur des kératinocytes préalablement exposés à l'alphabenzopyrène et à la lumière bleue sur la libération de l'IL-31 en pg/ml ;
- la figure 4 représente les concentrations en IL31, normalisé par la quantité de protéines totales cellulaires, illustrant les effets de l'asthaxanthine seule comparés aux effets de la myricitrine seule, après exposition de kératinocytes à de la lumière bleue et du benzo-a-pyrène ;
- la figure 5 représente la valeur d'inhibition du relargage d'IL31 pour deux concentrations en myricitrine et astaxanthine.

EXEMPLES

Exemple 1 : Culture des kératinocytes humains

Les cellules utilisées dans cette étude sont des cultures primaires de kératinocytes humains normaux (KHN) extraits après chirurgie cutanée d'un donneur de 30 ans.

Elles ont été cultivées dans un milieu complet KSFM: Keratinocyte-SFM (17005-31, Gibco) avec L-Glutamine, supplémenté avec un facteur de croissance épidermique recombinant humain (EGFhr, 10450-013,Gibco), extrait d'hypophyse bovine (BPE, 13028-014, Gibco) et 1% de pénicilline streptomycine (15070-063,Gibco) à 37 °C sous atmosphère de 5% de $CO_2$ jusqu'à 80% de confluence.

Exemple 2 : Test de viabilité des kératinocytes

A J0, les kératinocytes humains normaux sont incubés dans des plaques 96 puits dans un milieu de culture. Les cellules sont laissées en stabilisation pendant 24H à 37°C en atmosphère plus 5% de $CO_2$, comme décrit dans l'exemple 1.

A J1, les cellules sont traitées avec de la myricitrine, solubilisée dans le milieu de culture, à différentes concentrations. La culture est maintenue pendant 24 heures à 37°C et 5% de $CO_2$. La viabilité de KHN a été évaluée à l'aide du kit de prolifération cellulaire II (XTT) (11465015001, Roche) conformément aux instructions du fabricant.

Le système XTT, une méthode colorimétrique, est un test pour quantifier l'activité mitochondriale. Cette méthode, qui est simple, précise et permet des résultats reproductibles, peut être utilisée comme test de viabilité. Ce test est basé sur le clivage du sel de tétrazolium jaune XTT en formazan orange, par le système «succinate-tétrazolium réductase» présent dans la chaîne respiratoire mitochondriale des cellules. Ainsi, la conversion se produit uniquement dans les cellules métaboliquement actives, ce qui signifie des cellules vivantes.

Le formazan dérivé est mesuré par spectrophotométrie (à 450 nm avec 650 nm de référence). Pour chaque condition, des moyennes de données de densité optique (DO, absorbance) sont calculées.

La viabilité des cellules traitées est exprimée en pourcentage de contrôle non traité: un traitement qui diminue la viabilité des cellules, en dessous de la limite de 80% d'activité mitochondriale comparée chez le témoin non traité, est considéré comme cytotoxique pour les cellules. Au contraire, une augmentation des données est un signe d'activité mitochondriale et peut-être même un signe de prolifération cellulaire. Les kératinocytes cultivés selon l'exemple 1 sont exposés à une gamme de myricitrine allant de 0,21 à 50 µM. Le contrôle Témoin représente le milieu de culture sans adjuvant. Le contrôle+DMSO 10% représente un milieu toxique, témoin d'une toxicité cellulaire. L'expérience montre que la myricitrine n'a aucun effet toxique sur les kératinocytes.

Exemple 3 : Utilisation d'un flavonoïde, de type flavonol rhamnosylé, sur des kératinocytes en culture

Les kératinocytes cultivés selon l'exemple 1, sont exposés soit à de l'alphabenzopyrène (B[a]P) à 20 µM), soit à de la lumière bleue (450 nm) à 40J/$cm^2$, soit à la combinaison des deux aux mêmes doses. Un traitement à la myricitrine en quantité allant de 10 µM à 50 µM est appliqué à des cellules ayant préalablement reçu la combinaison B[a]P et lumière bleue.

Après 24H de traitement, un dosage de l'interleukine 31 (IL-31) relarguée par les cellules est effectué, via un kit d'immunodosage (ELISA). Le dosage d'IL-31 a été réalisé avec l'IL-31 humain ELISA MAXTM Deluxe Set (445704, Biolegend).

La combinaison d'alphabenzopyrène et lumière bleue induit une augmentation d'IL-31 par les kératinocytes de +111 %. L'expérience montre que l'ajout de myricitrine dès 20 µM induit une diminution de la production d'IL-31 de -100% sur les cellules prétraitées avec du B[a]P 20µM et de la lumière bleue à 40J/$cm^2$.

Exemple 4 : Utilisation d'un flavonoïde, de type flavonol rhamnosylé chez des patients Sur un panel de volontaires travaillant en milieu urbain [femmes âgées de 20 à 60 ans de type caucasien et asiatique] ayant des problèmes d'inconfort cutané sur le visage (démangeaisons, prurit, ...) est appliqué la crème de l'exemple 5 en hémi-visage et sur l'autre partie du visage une crème placébo sans la myricitrine.

Dès l'application et pendant huit jours d'usage matin et soir, les volontaires donnent leurs sensations organoleptiques-fraicheur, douceur et facilité d'application des produits, ainsi que les sensations physiologiques de type calme, arrêt des démangeaisons, via un questionnaire à remplir. Des analyses statistiques sont effectuées à la fin du test pour démontrer l'efficacité du produit contenant la myricitrine.

Exemple 5 : Crème calmante et adoucissante pour peaux urbaines ou à tendance prurigineuse.

A-Phase aqueuse

Glycérine 2.0%, Hexylene glycol 3.0%, Gomme de xanthane 0.5%, Myricitrine 0,5%, Conservateurs qs (quantité suffisante), Carbomer 0.35%, NaOH 0.35%, Eau qsp.100%

B-Phase Grasse

Squalane 15%, Alcool cétylique 2%, Arachidyl alcohol/behenyl alcohol/arachidylglucoside 1%, Stéarate de glycerol 5%, Conservateur et parfum qs

Exemple 6: Crème hydratante apaisante destinée à des peaux urbaines à tendance atopique

A-Phase Grasse

Ceteareth-2 3,5%, Ceteareth-21 entre 2 à 4%, Huile de germe de blé 3%, Cyclomethicone 7%, Octyl Palmitate 8%

B-Phase aqueuse

Glycérine 7.0%, Hexylene glycol 3.0%, Conservateurs qs, Eau qsp.100%

C-Ingrédients ajoutés dans l'émulsion, à une température inférieure à 40°C

Hyaluronate de sodium 0,1%, Myricitrine 1,0%, Eau 5%, Tocopherol 0,05%, Palmitate de vitamine A 0,1%, Phospholipides 0,5%, Céramides 3 0,1%, Polyacrylamide & C14-13 isoparaffine & laureth-7entre 2 à 3,5%

Exemple 7: Crème et lait destinés aux peaux exposées à la lumière bleue ou présentant des taches pigmentaires.

A-Phase Grasse

Monostéreate de Glycerol 2%, PEG-100 stearate 3%, C12-C15 alkyl benzoate 10%, Dimethicone 5%, Acétate de tocophérol 1%, Octyl-triazone (Uvinul T150) 1.5%, Butyl Methoxy Dibenzoyl methane (Eusolex 9020) 2.0 %, Alcool cetostéarylique 1%

B-Phase aqueuse

Eau qsp.100%, Quercitrine 0,5%, Conservateurs 0.6%, Glycérine 7%, Hexylene glycol 3.0%, Carbomer 0.5%, Tetra sodium EDTA 0,2%, Hyaluronate de sodium 0,1%, NaOH 0.5%, Conservateur + parfum qs.

C-Ingrédients ajoutés dans l'émulsion, à une température inférieure à 40°C

Acétate de tocophérol entre 0,1 à 1%, Pyridoxine entre 0,01 à 0,05%, Palmitate de vitamine A entre 0,01 à 1%, d-Panthénol entre 0,1 à 1%, Acide citrique entre 0,1 à 0,5%, Gluconate de zinc entre 0,1 à 1%, Citrate trisodique entre 1 à 2,5 %, Eau 5%.

Exemple 8: Brume protectrice destinée aux peaux exposées à la lumière bleue des appareils électroniques

Eau qsp. 100 %, Kaempferitrine 2%, Conservateurs 0.6%, Glycérine 7 %, Hexylene glycol 3.0%, Carbomer 0.5%, Tetra sodium EDTA 0,2%, Hyaluronate de sodium 0,1%, NaOH 0.5%, Conservateur + parfum qs.

Exemple 9 : comparaison des effets de l'astaxanthine et de la myricitrine sur le relargage d'IL31 par des kératinocytes primaires exposés aux effets combinés de la lumière bleue et du benzo-a-pyrène (BaP).

L'astaxanthine (dihydroxy-3,3' dioxo-4,4' β-carotène) est un caroténoïde, notamment synthétisé par des microalgues, cette molécule absorbant les longueurs d'ondes comprises entre 400 nm et 500 nm.

Le benzo-a-pyrène (CAS 50-32-8) est un des HAP (hydrocarbure aromatique polycyclique) les plus toxiques.

Les traitements des cellules ont été effectués de la manière suivante.

a) Type cellulaire : kératinocytes primaires d'épiderme humain (donneur 39 ans, ref : KER110, lot KER110049, Biopredic)

b) Conditions de culture : plaque 96-puits, 37°C, 5% $CO_2$, ensemencement 20.000 cellules/cm$^2$

c) Milieu de culture : KSFM (Gibco ref : 10144892) + 1% pénicilline/streptomycine

d) Traitement avec les composés : effectué à 24h de l'ensemencement,

e) Temps d'incubation avec les composés : 24 heures de contact

f) Concentrations des composés testées : myricitrine et astaxanthine à 20$\mu$M et 50$\mu$M

g) Condition de stress : effectuée à la suite des traitements avec les composés ; exposition à l'irradiation (lumière

bleue ; 40J/ cm$^2$ ; 450 nm) ; suivie par 24 heures de traitement avec 20$\mu$M benzo-a-pyrène (CRM40071, Sigma-Aldrich) en milieu de culture.

Les analyses ont été effectuées selon la procédure suivante.

- Lot Contrôle : aucun traitement en dehors du renouvellement du milieu de culture
- Prélèvements :

    Milieux de culture
    Culots cellulaires

- End-points / read-out :

    Dosage IL-31, à partir des milieux de culture (Human IL-31 ELISA MAX Deluxe ; Biolegends)
    Dosage des protéines totales, à partir des culots cellulaires (Méthode de Bradford)

Résultats : Dosage d'IL31

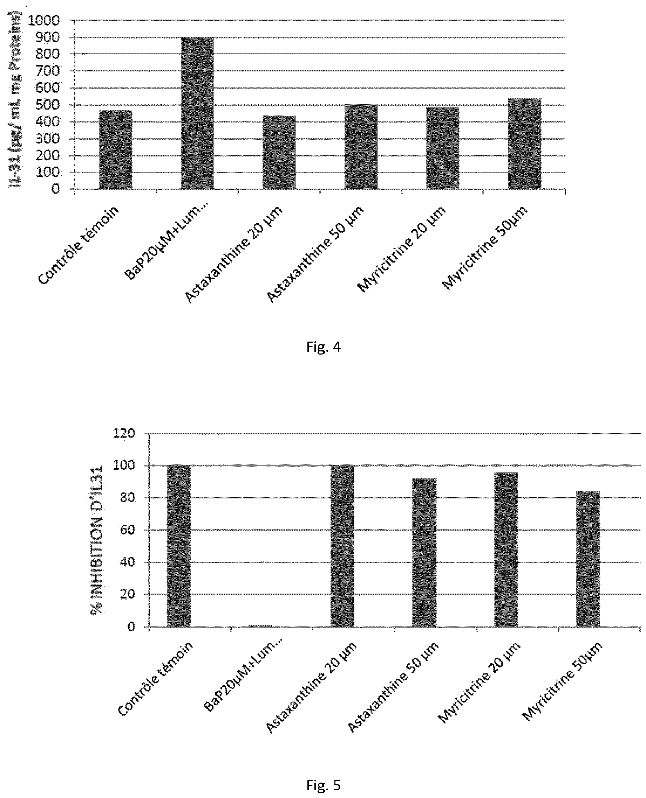

En figure 4, les résultats sont exprimés comme concentration en IL-31 normalisée par la quantité de protéines totales cellulaires.

En figure 5, une valeur d'inhibition du relargage (%) a été obtenue pour les groupes expérimentaux, conformément à la formule suivante :

$$\% \text{ Inhibition Relargage IL31 : } \% \text{ LotX} = (\text{IL31 Stress} - \text{IL31 Lot X})/(\text{IL31 Stress} - \text{IL31 Control}) \times 100$$

Comme référence, le groupe témoin a été considéré à efficacité maximale (100%) et le groupe de stress (Lum. Bleue + BaP) à l'efficacité minimale (à 0%):

## Revendications

1. Composition cosmétique et/ou dermatologique comprenant en tant qu'ingrédient actif au moins un flavonoïde glycosylé, **caractérisée en ce qu'**elle comprend en outre, en tant qu'ingrédient actif un composé capable de limiter ou d'empêcher la pénétration dans les kératinocytes d'un hydrocarbure aromatique polycyclique de type benzo[a]pyrene, cyclopenta[c,d]pyrène, dibenzo[a,h]anthracène ou dibenzo[a,l]pyrène et/ou un composé **caractérisé par** sa capacité à absorber la lumière bleue.

2. Composition cosmétique et/ou dermatologique selon la revendication 1, **caractérisée en ce que** ledit flavonoïde glycosylé est un flavonol glycosylé.

3. Composition cosmétique et/ou dermatologique selon la revendication 1 ou 2, **caractérisée en ce que** ledit flavonoïde glycosylé est rhamnosylé ou glucosylé, de préférence rhamnosylé.

4. Composition cosmétique et/ou dermatologique selon la revendication 3, **caractérisée en ce que** ledit flavonoïde rhamnosylé est choisi parmi la myricitrine, la quercitrine, la kaempferitrine, l'azaleine, l'icariine ou un mélange d'au moins deux de ces composés.

5. Composition cosmétique et/ou dermatologique selon la revendication 4, **caractérisée en ce que** ledit flavonoïde rhamnosylé est de la myricitrine.

6. Composition cosmétique et/ou dermatologique selon l'une quelconque des revendications précédentes, **caractérisée en ce que** ledit composé capable de limiter ou d'empêcher la pénétration dans les kératinocytes d'un hydrocarbure aromatique polycyclique de type benzo[a]pyrène, cyclopenta[c,d]pyrène, dibenzo[a,h]anthracène ou dibenzo[a,l]pyrène est choisi parmi les polysaccharides, les polysaccharides cycliques de type cyclodextrine, les émulsifiants, l'acide hyaluronique lipophilisé.

7.  Composition cosmétique et/ou dermatologique selon l'une quelconque des revendications précédentes, **caractérisée en ce que** ledit composé apte à absorber la lumière bleue est choisi parmi les caroténoïdes ou les mélanoïdines.

8.  Composition cosmétique et/ou dermatologique selon l'une quelconque des revendications précédentes, **caractérisée en ce qu'**elle est formulée pour une application topique.

9.  Composé flavonoïde glycosylé, de préférence un flavonol rhamnosylé, pour son utilisation dans le traitement d'une pathologie non allergique engendrant la production d'IL31, en particulier une pathologie affectant la peau et choisie parmi le prurit, la dermatite atopique, l'eczéma, le psoriasis, le prurigo nodulaire.

10. Composé flavonoïde glycosylé, de préférence un flavonol rhamnosylé selon la revendication 9, choisi parmi la myricitrine, la quercitrine, la kaempferitrine, l'azaleine, l'icariine ou un mélange d'au moins deux de ces composés.

Fig. 1

Fig. 2

Fig. 3

Fig. 4

Fig. 5

# EP 3 957 306 A1

Europäisches
Patentamt

European
Patent Office

Office européen
des brevets

## RAPPORT DE RECHERCHE EUROPEENNE

Numéro de la demande

EP 21 18 3515

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | MA 32 836 B1 (FABRE PIERRE DERMO COSMETIQUE [FR]) 1 novembre 2011 (2011-11-01) * revendications 1,2,15 * ----- | 1-10 | INV. A61K31/015 A61K31/366 A61K31/7048 A61K31/715 |
| T | WO 2020/225319 A1 (PF MEDICAMENT [FR]) 12 novembre 2020 (2020-11-12) * revendications 1,12 * ----- | | A61K31/724 A61K31/728 A61P17/04 A61P17/06 |
| X | US 9 498 505 B2 (BAYER CONSUMER CARE AG [CH]) 22 novembre 2016 (2016-11-22) * revendications 1,2,7,8 * ----- | 9,10 | A61K8/60 A61Q19/00 A61K8/73 A61Q17/00 |
| X | US 2014/086977 A1 (O'NEILL CATHERINE ANNE [GB] ET AL) 27 mars 2014 (2014-03-27) * revendications 1-9 * ----- | 9,10 | |
| X | CN 106 138 077 A (HUASHAN HOSPITAL FUDAN UNIV) 23 novembre 2016 (2016-11-23) * abrégé * ----- | 9,10 | |
| X | JP H07 300581 A (PROD DEV RES INST) 14 novembre 1995 (1995-11-14) | 1-4,7 | DOMAINES TECHNIQUES RECHERCHES (IPC) A61K |
| Y | * abrégé * ----- | 1-10 | A61P A61Q |
| X | PROTTI MICHELE ET AL: "Analytical profiling of selected antioxidants and total antioxidant capacity of goji (Lyciumspp.) berries", JOURNAL OF PHARMACEUTICAL AND BIOMEDICAL ANALYSIS, ELSEVIER B.V, AMSTERDAM, NL, vol. 143, 3 juin 2017 (2017-06-03), pages 252-260, XP085108203, ISSN: 0731-7085, DOI: 10.1016/J.JPBA.2017.05.048 * tableau 4 * ----- -/-- | 1-7 | |

Le présent rapport a été établi pour toutes les revendications

| Lieu de la recherche | Date d'achèvement de la recherche | Examinateur |
|---|---|---|
| La Haye | 14 décembre 2021 | Bonzano, Camilla |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........................................................................................
& : membre de la même famille, document correspondant

EPO FORM 1503 03.82 (P04C02)

page 1 de 2

Europäisches
Patentamt

European
Patent Office

Office européen
des brevets

# RAPPORT DE RECHERCHE EUROPEENNE

Numéro de la demande

EP 21 18 3515

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | CN 105 342 975 A (QINGDAO HUAXIA HEAVY INDUSTRY CO LTD) 24 février 2016 (2016-02-24) * abrégé * ----- | 1-8 | |
| X | US 2019/374591 A1 (OSORIO KAREN MARIE [US] ET AL) 12 décembre 2019 (2019-12-12) * alinéa [0043] * ----- | 1-10 | |
| X | DATABASE GNPD [Online] MINTEL; 24 mars 2020 (2020-03-24), anonymous: "SD Advanced Plus Intensive Moisturizing Concentrate", XP55872965, Database accession no. 7466277 * abrégé * ----- | 1-8 | |

DOMAINES TECHNIQUES
RECHERCHES (IPC)

Le présent rapport a été établi pour toutes les revendications

| Lieu de la recherche | Date d'achèvement de la recherche | Examinateur |
|---|---|---|
| La Haye | 14 décembre 2021 | Bonzano, Camilla |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

**EP 21 18 3515**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**14-12-2021**

| Document brevet cité au rapport de recherche | | | Date de publication | Membre(s) de la famille de brevet(s) | | | Date de publication |
|---|---|---|---|---|---|---|---|
| MA 32836 | B1 | | 01-11-2011 | AR | 073296 | A1 | 28-10-2010 |
| | | | | AU | 2009315858 | A1 | 30-06-2011 |
| | | | | BR | PI0921042 | A2 | 01-09-2020 |
| | | | | CA | 2742927 | A1 | 20-05-2010 |
| | | | | CN | 102215854 | A | 12-10-2011 |
| | | | | CO | 6361940 | A2 | 20-01-2012 |
| | | | | DK | 2365819 | T3 | 11-06-2019 |
| | | | | EP | 2365819 | A2 | 21-09-2011 |
| | | | | ES | 2731455 | T3 | 15-11-2019 |
| | | | | FR | 2938439 | A1 | 21-05-2010 |
| | | | | GE | P20156374 | B | 12-10-2015 |
| | | | | HK | 1157653 | A1 | 06-07-2012 |
| | | | | IL | 212863 | A | 30-03-2017 |
| | | | | JP | 5693459 | B2 | 01-04-2015 |
| | | | | JP | 2012508708 | A | 12-04-2012 |
| | | | | KR | 20110086138 | A | 27-07-2011 |
| | | | | KR | 20160103181 | A | 31-08-2016 |
| | | | | MA | 32836 | B1 | 01-11-2011 |
| | | | | MY | 162341 | A | 15-06-2017 |
| | | | | NZ | 593264 | A | 28-03-2013 |
| | | | | PL | 2365819 | T3 | 31-01-2020 |
| | | | | PT | 2365819 | T | 12-06-2019 |
| | | | | RU | 2011121437 | A | 20-12-2012 |
| | | | | TN | 2011000219 | A1 | 17-12-2012 |
| | | | | TR | 201906952 | T4 | 21-06-2019 |
| | | | | TW | 201018476 | A | 16-05-2010 |
| | | | | UA | 107653 | C2 | 10-02-2015 |
| | | | | US | 2011217398 | A1 | 08-09-2011 |
| | | | | US | 2016228486 | A1 | 11-08-2016 |
| | | | | WO | 2010054879 | A2 | 20-05-2010 |
| | | | | ZA | 201104056 | B | 25-01-2012 |
| WO 2020225319 | A1 | | 12-11-2020 | FR | 3095759 | A1 | 13-11-2020 |
| | | | | WO | 2020225319 | A1 | 12-11-2020 |
| US 9498505 | B2 | | 22-11-2016 | CA | 2869896 | A1 | 17-10-2013 |
| | | | | EP | 2649987 | A1 | 16-10-2013 |
| | | | | EP | 2838504 | A2 | 25-02-2015 |
| | | | | JP | 2015512922 | A | 30-04-2015 |
| | | | | KR | 20150003193 | A | 08-01-2015 |
| | | | | US | 2015086662 | A1 | 26-03-2015 |
| | | | | US | 2017042956 | A1 | 16-02-2017 |
| | | | | WO | 2013153108 | A2 | 17-10-2013 |
| US 2014086977 | A1 | | 27-03-2014 | EP | 2691090 | A2 | 05-02-2014 |
| | | | | US | 2014086977 | A1 | 27-03-2014 |

EPO FORM P0460

Pour tout renseignement concernant cette annexe : voir Journal Officiel de l'Office européen des brevets, No.12/82

**page 1 de 2**

**ANNEXE AU RAPPORT DE RECHERCHE EUROPEENNE
RELATIF A LA DEMANDE DE BREVET EUROPEEN NO.**

**EP 21 18 3515**

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

**14-12-2021**

| Document brevet cité au rapport de recherche | Date de publication | Membre(s) de la famille de brevet(s) | Date de publication |
|---|---|---|---|
| | | WO    2012131341 A2 | 04-10-2012 |
| CN 106138077    A | 23-11-2016 | AUCUN | |
| JP H07300581    A | 14-11-1995 | AUCUN | |
| CN 105342975    A | 24-02-2016 | AUCUN | |
| US 2019374591    A1 | 12-12-2019 | CN     112118856 A<br>EP       3801588 A1<br>US     2019374591 A1<br>US     2021205386 A1<br>WO     2019236935 A1 | 22-12-2020<br>14-04-2021<br>12-12-2019<br>08-07-2021<br>12-12-2019 |

EPO FORM P0460

Pour tout renseignement concernant cette annexe : voir Journal Officiel de l'Office européen des brevets, No.12/82

**page 2 de 2**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 10273297 B
- EP 2734549 A

**Littérature non-brevet citée dans la description**

- **KRUTMANN J. et al.** The skin aging exposome. *Dermatol Sci.,* Mars 2017, vol. 85 (3), 152-161
- **DUTEIL et al.** Differences in visible light-induced pigmentation according to wavelengths: a clinical and histological study in comparison with UVB exposure. *Pigment Cell Melanoma Res,* 2014, vol. 27 (5), 822-6 T
- **ANNE VON KOSCHEMBAHR et al.** Metabolism and genotoxicity of polycyclic aromatic hydrocarbons in human skin explants: mixture effects and modulation by sunlight. *Archives of Toxicology,* 2020, vol. 94, 495-507
- **ANNE VON KOSCHEMBAHR et al.** Solar simulated light exposure alters metabolization and genotoxicity induced by benzo[a]pyrene in human skin. *Scientific Reports,* 2018, vol. 8
- **AYAKO TAKAMORI et al.** IL-31 is crucial for induction of pruritus, but not inflammation, in contact hypersensitivity. *Sci Rep,* 2018, vol. 8, 6639
- **YANG J et al.** The influence of PM2.5 on lung injury and cytokines in mice. *Exp Ther Med.,* Octobre 2019, vol. 18 (4), 2503-2511
- **WANG Y. et al.** *Mech Ageing Dev.,* Décembre 2019, vol. 184, 111160
- **VANDERSEE S. et al.** Blue-Violet Light Irradiation Dose Dependently Decreases Carotenoids in Human Skin, Which Indicates the Génération of Free Radicals. *Oxidative Med Cell Longev.,* vol. 2015
- **MOREIRA AS et al.** Coffee melanoidins: structures, mechanisms of formation and potential health impacts. *Food Funct.,* Septembre 2012, vol. 3 (9), 903-15
- **MENG J.** New mechanism underlying IL-31-induced atopic dermatitis. *J Allergy Clin Immunol.,* Mai 2018, vol. 141 (5), 1677-1689
- **BAĞCI IS et al.** IL-31: A new key player in dermatology and beyond. *J Allergy Clin Immunol.,* Mars 2018, vol. 141 (3), 858-866
- *CHEMICAL ABSTRACTS,* 50-32-8